# EUROPÄISCHE PATENTSCHRIFT

(11) **EP 3 082 883 B1**
(45) Veröffentlichungstag und Bekanntmachung des Hinweises auf die Patenterteilung: **25.10.2017**
(21) Anmeldenummer: 14816168.0
(22) Anmeldetag: 09.12.2014
(51) Int. Cl.: A61L 2/14, A61F 2/00

(54) **PLASMAANLAGE MIT EINEM SEPARAT TRANSPORTIERBAREN GEFÄSS**
PLASMA SYSTEM WITH A SEPARATELY TRANSPORTABLE VESSEL
INSTALLATION À PLASMA COMPORTANT UN RÉCIPIENT TRANSPORTABLE SÉPARÉMENT

(30) Priorität: 20.12.2013 DE 102013226814
(43) Veröffentlichungstag der Anmeldung: 26.10.2016
(73) Patentinhaber: Diener, Christof-Herbert, 72202 Nagold (DE)
(72) Erfinder: DIENER, Christof-Herbert, 72202 Nagold (DE)
(74) Vertreter: Kohler Schmid Möbus Patentanwälte
(86) Internationale Anmeldenummer: PCT/EP2014/077029
(87) Internationale Veröffentlichungsnummer: WO 2015/091104

(56) Entgegenhaltungen:
- WO-A1-2013/101673
- WO-A1-2014/132216
- WO-A2-2011/116984
- US-A- 3 383 163
- US-A1- 2013 118 406

## Beschreibung

Die Erfindung betrifft eine Plasmaanlage zur Behandlung eines Gegenstandes, insbesondere eines Implantates, wobei die Plasmaanlage ein verschließbares Gefäß zur Aufnahme des Gegenstandes und eine Plasmaeinheit mit einer elektrischen Plasmaquelle zur Erzeugung eines Plasmas in dem Gefäß aufweist, wobei das Gefäß als von der Plasmaeinheit unabhängig transportierbare Einheit ausgebildet ist, wobei das Plasma in dem Gefäß unmittelbar durch die elektrische Plasmaquelle der Plasmaeinheit erzeugbar ist und das Gefäß einen Gefäßkörper aufweist, der im Wesentlichen aus einem dielektrischen Material ausgebildet ist.

US3383163 offenbart eine Vorrichtung zur Plasmasterilisation von Gläsern gemäß dem Oberbegriff des Anspruchs 1.

Es ist bekannt, einen Gegenstand in einem Plasma zu behandeln. Weiterhin ist es bekannt, Implantate, insbesondere Zahnimplantate, kurz vor deren Implantation in einem Plasma zu behandeln. Ein solches Plasmabehandlungsverfahren ist beispielsweise aus der DE 195 02 133 A1 bekannt geworden.

Das Abtöten selbst multiresistenter Keime in einem Plasma wird von Peter Trechow in den VDI-Nachrichten vom 22.11.2013, Ausgabe 47 beschrieben. Dabei wird ausgeführt, dass es durch plasmabasierte Oberflächenfunktionalisierung von Implantaten möglich ist, diese selektiv für Zellen besonders attraktiv zu machen und damit ein Einheilen permanenter Implantate zu beschleunigen. Die Anwendung eines Plasmas auf Implantate ermöglicht somit, zellattraktive mit antimikrobiellen Eigenschaften zu kombinieren.

Das Reinigen von Implantaten in einem Plasma ist weiterhin aus dem Artikel "Microscopical an microbiologic characterization of customized titanium abutments after different cleaning procedures", Clinical Oral Implants Research, 0, 2012/1-9, bekannt geworden.

Die US 2013/0230426 A1 offenbart eine Plasmaanlage zur Sterilisation von Implantaten in einem Vakuumbehälter. Der Vakuumbehälter ist mit einer Pumpeneinheit verbunden, um einen Unterdruck im Behälter während der Plasmabehandlung zu erzeugen. Eine solche Vorrichtung zur Plasma-Sterilisation ist weiterhin aus der WO 20101/044669 A1 bekannt geworden.

Die Plasmabehandlung des Implantates sollte zur Erzielung bestmöglicher Wirkung unmittelbar vor dem Implantieren erfolgen. Hierzu ist es bislang nötig, aufwändige und teure Plasmaanlagen in Arztpraxen bzw. Krankenhäusern zu installieren. Diese Plasmaanlagen müssen in der Regel mit einer Pumpe und mehreren Gasflaschen verbunden sein, wodurch sich Aufwand und Kosten für die Plasmaanlage erhöhen. Darüber hinaus müssen solche Plasmaanlagen von speziell geschultem Personal bedient werden.

Aus der DE 10 2004 049 783 B4 und der WO 03/059400 A1 ist es demgegenüber bekannt geworden, eine Plasmaanlagen in eine Plasmaeinheit mit einer elektrischen Plasmaquelle und ein von der Plasmaeinheit unabhängig transportierbares Gefäß aufzuteilen.

Die bekannten Gefäße müssen jedoch aufwändig mit Elektroden versehen werden. Diese Elektroden müssen zur Plasmabehandlung des Gegenstandes kontaktiert werden. Die Ausbildung der Gefäße ist dadurch kostenintensiv. Weiterhin muss die Kontaktierung der Gefäße durch geschultes Personal vorgenommen werden.

Aus der WO 2011/116984 A2 ist eine Plasmaanlage mit einem Gefäß bekannt geworden, wobei das Gefäß keine Elektroden zum Zünden eines Plasmas aufweist. Zum Zünden des Plasmas muss das Gefäß jedoch in unmittelbaren Kontakt mit einer Elektrodenstruktur der Plasmaanlage gebracht werden.

Die US 6,558,621 B1 offenbart, ein Implantat unmittelbar nach einer Plasmabehandlung in einem Gefäß einzuschließen und aus der Plasmabehandlungskammer auszuschleusen.

Weiterhin ist es aus der WO 97/44503 A1 bekannt geworden, in einer Plasmakammer die Innenseite eines Behälters mittels einer Plasmabehandlung zu sterilisieren.

Aufgabe der vorliegenden Erfindung ist es demgegenüber, eine Plasmaanlage bereitzustellen, mit der Gegenstände, insbesondere Implantate, einfach und kostengünstig plasmabehandelbar sind.

Diese Aufgabe wird erfindungsgemäß durch eine Plasmaanlage mit den Merkmalen des Patentanspruchs 1 gelöst. Die Patentansprüche 2 bis 6 geben bevorzugte Weiterbildungen einer solchen Plasmaanlage an.

Die Plasmaanlage zur Behandlung eines Gegenstandes, insbesondere eines Implantates, umfasst ein verschließbares Gefäß zur Aufnahme des Gegenstandes und eine Plasmaeinheit mit einer elektrischen Plasmaquelle zur Erzeugung eines Plasmas in dem Gefäß, wobei das Gefäß als von der Plasmaeinheit unabhängig transportierbare Einheit ausgebildet ist und das Plasma unmittelbar durch die elektrische Plasmaquelle der Plasmaeinheit erzeugbar ist, wobei das Gefäß einen Gefäßkörper aufweist, der im Wesentlichen aus einem dielektrischen Material ausgebildet ist, wobei die Plasmaeinheit keinen Gasanschluss und/oder keine Pumpe aufweist und derart ausgebildet ist, dass das Plasma zündbar ist, wenn sich das Gefäß distanziert zur Plasmaeinheit befindet.

Die Plasmaanlage umfasst somit ein eigenständig transportierbares, verschließbares Gefäß. In dem Gefäß kann der Gegenstand, insbesondere das Implantat, aufgenommen werden. Der Hersteller des Gegenstandes kann daher den Gegenstand in dem Gefäß verschließen und ausliefern. Unter dem Begriff "eigenständig transportierbares Gefäß" wird dabei ein Gefäß verstanden, das nicht permanent mit der Plasmaeinheit verbunden ist. Insbesondere ist das Gefäß nicht über Schläuche für Gase und/oder elektrische Leitungen dauerhaft mit der Plasmaeinheit verbunden.

Unmittelbar vor Verwendung des Gegenstandes kann das Gefäß direkt am Einsatzort in die Nähe der Plasmaeinheit gebracht werden. Die Plasmaeinheit erzeugt dann in dem Gefäß ein Plasma. Das Plasma behandelt den Gegenstand in dem Gefäß. Anschließend kann das Gefäß geöffnet, der Gegenstand aus dem Gefäß entnommen und eingesetzt (insbesondere implantiert) werden. Die Plasmaeinheit muss dadurch lediglich eine elektrische Baugruppe zur Erzeugung des Plasmas, nämlich die elektrische Plasmaquelle, aufweisen. Die Plasmaeinheit kann dadurch besonders einfach und kostengünstig ausgebildet sein.

Die elektrische Plasmaquelle erzeugt das Plasma unmittelbar in dem Gefäß. Unter dieser unmittelbaren Erzeugung des Plasmas wird dabei eine "Fernwirkung" der elektrischen Plasmaquelle auf das Gefäß verstanden. Das Gefäß kann dadurch elektrodenfrei ausgebildet sein. Hierdurch werden die Kosten zur Herstellung des Gefäßes deutlich verringert, zumal Elektroden zum Zünden des Plasmas in genau definiertem Abstand in dem Gefäß ausgebildet werden müssen. Die Plasmaeinheit ist dabei derart ausgebildet, dass das Plasma zündbar ist, wenn sich das Gefäß distanziert zur Plasmaeinheit befindet. Das Gefäß muss somit nur in die Nähe der Plasmaeinheit gebracht werden, um ein Plasma in dem Gefäß zu zünden. Eine Kontaktierung des Gefäßes durch geschultes Personal entfällt hierdurch.

Das Gefäß ist besonders bevorzugt dauerhaft luftdicht verschließbar.

Das Gefäß kann mit einer Schnellkupplung, beispielsweise von Cajon oder Swagelok, versehen sein, um eine schnelle und einfache Druckeinstellung in dem Gefäß zu ermöglichen.

Besonders bevorzugt ist das Gefäß mit einem irreversiblen Temperaturindikator versehen, um überprüfen zu können, ob der Gegenstand in dem Gefäß bereits plasmabehandelt wurde. Der Temperaturindikator ist dabei vorzugsweise zum Einsatz in einem Temperaturbereich zwischen 30°C und 100°C, vorzugsweise zwischen 40°C und 60°C ausgebildet. Durch einen solchen Temperaturindikator kann weiterhin beobachtet werden, ob die nötige Zeitdauer der Plasmabehandlung bereits erreicht wurde.

Die Plasmaeinheit weist keinen Gasanschluss und/oder keine Pumpe auf. Hierdurch ist die Plasmaeinheit besonders einfach ausgebildet. Weiterhin ist eine Plasmaeinheit ohne Medienanschlüsse besonders leicht bedienbar.

Die Plasmaanlage ist derart ausgebildet, dass die Plasmaeinheit, insbesondere während der Plasmabehandlung, keine Verbindung zum Gefäß aufweist. Mit anderen Worten besteht weder eine elektrische, noch eine fluidische Leitung zwischen Gefäß und Plasmaeinheit. Bevorzugt besteht auch keine feste mechanische Verbindung zwischen Plasmaeinheit und Gefäß. Die Plasmaeinheit weist bevorzugt keine abgeschlossene Plasmakammer auf.

Die Plasmaeinheit weist besonders bevorzugt ein Gehäuse mit einer Ausnehmung zur Aufnahme des Gefäßes auf, wobei das Gehäuse das Gefäß nur teilweise umschließt.

Der Gefäßkörper kann aus Keramik oder einem resistenten Kunststoff ausgebildet sein. Besonders bevorzugt ist der Gefäßkörper vollständig aus einem dielektrischen Material ausgebildet.

In besonders bevorzugter Ausgestaltung der Erfindung ist der Gefäßkörper zumindest teilweise, insbesondere im Wesentlichen, vorzugsweise vollständig, aus Glas ausgebildet. Glas ist transparent, so dass ein Benutzer der Plasmaanlage das Zünden des Plasmas im Gefäß beobachten kann und darüber hinaus besonders plasmaresistent.

Das Gefäß kann aus einem Metallrohr mit einer Glasscheibe ausgebildet sein.

Der Gefäßkörper ist dabei vorzugsweise in Form einer Ampulle ausgebildet. Solche Ampullen eignen sich besonders gut zur Behandlung eines Implantates, da Ampullen in Arztpraxen und Kliniken bereits bekannt und weit verbreitet sind. Der Umgang mit Ampullen ist dem Personal in Kliniken und Arztpraxen daher bereits geläufig. Die Ampulle kann zumindest eine zugeschmolzene Spitze aufweisen.

Das Gefäß kann einen Stopfen zum Verschluss des Gefäßkörpers aufweisen. Gefäßkörper und Stopfen weisen dabei vorzugsweise jeweils einen Schliff auf, um den Gefäßkörper mit dem Stopfen luftdicht verschließen zu können.

In einer weiteren bevorzugten Ausführungsform der Erfindung ist das Gefäß nach Aufnahme des Gegenstandes nicht zerstörungsfrei öffenbar. Ein solches Gefäß kann einerseits besonders kostengünstig hergestellt werden und weist andererseits den Vorteil besonders hoher Dichtheit auf. Weiterhin ist bei einem solchen Gefäß sofort erkennbar, ob dieses noch "originalverpackt" vom Hersteller vorliegt oder bereits geöffnet bzw. manipuliert wurde. Der Gefäßkörper kann dabei einteilig ausgebildet sein und den Gegenstand vollständig umschließen.

Ein Gefäßkörper des Gefäßes ist dabei in Form einer Brechampulle ausgebildet. Unter einer Brechampulle wird eine Glasampulle verstanden, die zum Öffnen aufgebrochen werden muss. Die Brechampulle kann eine, insbesondere ringförmige, Sollbruchstelle aufweisen. Die Sollbruchstelle kann beispielsweise in den Gefäßkörper eingeritzt sein. Alternativ oder zusätzlich dazu kann eine Sollbruchstelle in Form eines eingebrannten Emaillerings ausgebildet sein.

Der Gefäßkörper ist zur einfachen Herstellung des Gefäßkörpers und zum leichten Einbringen in die Plasmaeinheit vorzugsweise im Wesentlichen rotationssymmetrisch zu seiner Längsachse ausgebildet. Besonders bevorzugt weist der Gefäßkörper dabei einen zylinderförmigen Abschnitt auf.

Der Gefäßkörper ist vorzugsweise einteilig und insbesondere derart ausgebildet, dass er einen darin aufgenommenen Gegenstand vollständig umschließt.

Um ein Niederdruckplasma im Gefäßkörper zünden zu können, kann der Gasdruck im Gefäßkörper weniger als 1 bar, insbesondere weniger als 0,1 bar, vorzugsweise weniger als 0,01 bar, besonders bevorzugt weniger als 0,001 bar betragen.

Die erfindungsgemäße Plasmaanlage kann dazu eingesetzt werden, eine Oberfläche des Gegenstandes in dem Gefäß anzuätzen. In dem Gefäß ist in diesem Fall vorzugsweise ein ätzendes Gas, beispielsweise Tetrafluormethan (CF₄), Hexafluorethan (C₂F₆), Perfluorpropan (C₃F₈), Oktafluorzyklobutan (C₄F₈), Schwefelhexafluorid (SF₆), und/oder Stickstofftrifluorid (NF₃) enthalten. Das Gefäß enthält bevorzugt ein fluorhaltiges Gas, um eine Titanoberfläche des Gegenstandes in dem Gefäß anzuätzen.

Das erfindungsgemäße Plasmabehandlungsverfahren kann weiterhin dazu eingesetzt werden, eine Oberfläche des Gegenstandes zu aktivieren. In dem Gefäß ist hierzu vorzugsweise Gas in Form von Argon (Ar), Sauerstoff (O₂) und/oder Luft enthalten. Alternativ oder zusätzlich dazu kann in dem Gefäß Wasser (H₂O), insbesondere in gasförmigem Zustand, enthalten sein.

Das Gefäß kann weiterhin ein aminhaltiges Gas, insbesondere Methylamin (CH₅N), Dimethylamin (C₂H₇N), Trimethylamin (C₃H₉N), Ammoniak (NH₃), Betain (C₅H₁₁NO₂) und/oder Butylamin (C₄H₁₁N), enthalten. Das aminhaltige Gas wird besonders bevorzugt im Falle eines Gegenstandes in Form eines Implantates eingesetzt, da sich gezeigt hat, dass biologische Zellen besonders gut auf Implantaten anwachsen, die mit einem aminhaltigen Gas plasmabehandelt wurden.

Das Befüllen des Gefäßes wird stark vereinfacht, wenn in dem Gefäß Atmosphärendruck, d.h. Umgebungsdruck nach dem Verschließen herrschen soll. Um trotz des Atmosphärendrucks ein Zünden des Plasmas zu ermöglichen, ist das Gefäß in diesem Fall zu mehr als 40%, insbesondere zu mehr als 50%, vorzugsweise zu mehr als 60%, besonders bevorzugt zu mehr als 70% mit Helium (He) gefüllt.

Das erfindungsgemäße Plasmabehandlungsverfahren kann weiterhin dazu eingesetzt werden, eine Schicht auf den Gegenstand in dem Gefäß aufzutragen. Hierzu kann das Gefäß eine im Plasma zerfallende Verbindung, beispielsweise eine Silanverbindung, enthalten.

Die elektrische Plasmaquelle der Plasmaeinheit kann zur unmittelbaren Plasmaerzeugung in dem Gefäß einen Hochfrequenzgenerator und mit dem Hochfrequenzgenerator elektrisch verbundene Elektroden aufweisen, zwischen die das Gefäß zumindest teilweise einbringbar ist. Die Elektroden sind dabei vorzugsweise im Wesentlichen ringförmig ausgebildet, um das Gefäß auf einfache Art und Weise zwischen die Elektroden einbringen zu können. Besonders bevorzugt sind die Elektroden um die Ausnehmung des Gehäuses der Plasmaeinheit ausgebildet, die zur Aufnahme des Gefäßes ausgebildet ist.

Zur unmittelbaren Erzeugung des Plasmas im Gefäß kann die elektrische Plasmaquelle ein Magnetron aufweisen, wobei das Gefäß zumindest teilweise von der Mikrowellenstrahlung des Magnetrons durchdringbar ist. Um die elektromagnetische Strahlung des Magnetrons zielgerichtet auf das Gefäß richten zu können, kann das Magnetron mit einer Antenne zur Abstrahlung der elektromagnetischen Strahlung verbunden sein. Die Antenne ist besonders bevorzugt um die Ausnehmung des Gehäuses der Plasmaeinheit ausgebildet, die zur Aufnahme des Gefäßes ausgebildet ist.

Die unmittelbare Erzeugung des Plasmas in dem Gefäß kann weiterhin erreicht werden, dass die elektrische Plasmaquelle eine Induktionsspule aufweist, in die das Gefäß zumindest teilweise einbringbar ist. Die Induktionsspule ist besonders bevorzugt um die Ausnehmung des Gehäuses der Plasmaeinheit ausgebildet, die zur Aufnahme des Gefäßes ausgebildet ist.

Die Erfindung betrifft weiterhin ein Verfahren zur Behandlung eines Gegenstandes, insbesondere eines Implantates, gemäß Patentanspruch 7.

Wie zuvor ausgeführt, bietet dieses erfindungsgemäße Verfahren den besonderen Vorteil, eine konstruktiv sehr einfach ausgebildete Plasmaeinheit beim Anwender des Gegenstandes, beispielsweise beim Verwender eines Implantates in einer Klinik oder einer Arztpraxis, einsetzen zu können. Der Gegenstand, insbesondere das Implantat, kann vom Hersteller des Gegenstandes in dem Gefäß zum Verwender des Gegenstandes geliefert werden. Im Falle eines Implantates kann die Plasmabehandlung kurz vor der Operation, d.h. maximal ca. 1 Stunde vor der Operation, erfolgen. Der Gegenstand, bzw. das Implantat, kann dann mit frisch beschichteter, geätzter und/oder aktivierter Oberfläche verwendet werden. Das Gefäß ist beim Zünden des Plasmas distanziert, d.h. beabstandet von der Plasmaeinheit. Das Gefäß muss dadurch nur ungefähr in die Nähe der Plasmaeinheit gebracht werden. Eine genaue Positionierung ist nicht nötig.

Die eingesetzte Plasmaanlage weist vorzugsweise eines oder mehrere der zuvor beschriebenen Merkmale auf.

Besonders bevorzugt wird ein Gefäßkörper aus Glas eingesetzt.

Bevorzugt wird ein Gefäßkörper eingesetzt, der einteilig ausgebildet ist und den Gegenstand insbesondere vollständig umschließt.

In besonders bevorzugter Ausgestaltung der Erfindung wird ein Gefäß eingesetzt, das nach Aufnahme des Gegenstandes nicht zerstörungsfrei öffenbar ist.

Im Verfahrensschritt b) wird vorzugsweise ein Gasdruck von weniger als 1 bar, insbesondere weniger als 0,1 bar, vorzugsweise weniger als 0,01 bar, besonders bevorzugt weniger als 0,001 bar eingestellt.

Zum Zünden des Plasmas kann eine elektrische Plasmaquelle in der Plasmaeinheit verwendet werden, die einen Hochfrequenzgenerator und mit dem Hochfrequenzgenerator elektrisch verbundene Elektroden aufweist, zwischen die das Gefäß zumindest teilweise eingebracht wird.

Zum unmittelbaren Zünden des Plasmas kann weiterhin eine elektrische Plasmaquelle eingesetzt werden, die ein Magnetron aufweist, wobei das Gefäß zumindest teilweise von der Mikrowellenstrahlung des Magnetrons durchdrungen wird.

Das erfindungsgemäße Verfahren wird besonders bevorzugt dazu eingesetzt, ein Implantat, insbesondere ein Zahnimplantat, kurz vor dem Implantieren zu aktivieren. Eine Verklebung des Implantates kann dadurch unter Vermeidung von Oberflächenanätzungen, insbesondere unter Vermeidung der Verwendung von Flusssäure (HF) erfolgen.

Vorzugsweise werden die Parameter zur Durchführung des erfindungsgemäßen Verfahrens so gewählt, dass sich die Oberflächenenergie des Gegenstandes zumindest verdoppelt. Versuche mit Gegenständen aus Titan haben gezeigt, dass sich die Oberflächenenergie von 43mN/m auf weit über 100mN/m bei der Durchführung des erfindungsgemäßen Verfahrens erhöhen lässt. Hierdurch wird eine besonders effiziente Aktivierung der Oberfläche des Gegenstandes erreicht.

Das erfindungsgemäße Verfahren wird besonders bevorzugt dazu eingesetzt, eine Plasmabehandlung durchzuführen, um ein Anwachsen eines Implantates zu verbessern. Dabei kommt es nicht darauf an, das Implantat im Plasma zu sterilisieren, sondern zu aktivieren. Das Implantat liegt somit bereits vor der Plasmabehandlung steril in dem Gefäß vor.

Die Erfindung betrifft schließlich gemäß Patentanspruch 8 die Verwendung eines elektrodenfrei ausgebildeten, unabhängig von einer Plasmaeinheit transportierbaren Gefäßes.

Das verwendete Gefäß weist vorzugsweise eines oder mehrere der zuvor beschriebenen Merkmale auf.

Das Gefäß wird besonders bevorzugt in einer Arztpraxis, insbesondere einer Zahnarztpraxis oder einer Klinik verwendet.

Ein besonders geeigneter Einsatz des Gefäßes besteht dabei darin, ein Implantat, insbesondere ein Zahnimplantat, kurz vor der Behandlung zu aktivieren, sodass es ohne weitere chemische Behandlung, insbesondere unter Vermeidung von Oberflächenanätzungen und dergleichen, verwendet werden kann.

Weitere Merkmale und Vorteile der Erfindung ergeben sich aus der nachfolgenden detaillierten Beschreibung mehrere Ausführungsbeispiele der Erfindung, anhand der Figuren der Zeichnung, die erfindungswesentliche Einzelheiten zeigt sowie aus den Patentansprüchen.

Die in der Zeichnung gezeigten Merkmale sind derart dargestellt, dass die erfindungsgemäßen Besonderheiten deutlich sichtbar gemacht werden können. Die verschiedenen Merkmale können je einzeln für sich oder zu mehreren in beliebigen Kombinationen bei Varianten der Erfindung verwirklicht sein.

Es zeigen:
- Figur 1: eine schematische Ansicht einer ersten Plasmaanlage;
- Figur 2: eine schematische Ansicht einer zweiten Plasmaanlage;
- Figur 3: eine schematische Ansicht einer dritten Plasmaanlage;
- Figur 4a: eine Plasmaanlage gemäß der US 6,558,621 B1;
- Figur 4b: im Vergleich zur Plasmaanlage gemäß Figur 4a eine erfindungsgemäße Plasmaanlage;
- Figur 5a: ein schematisches Druck-Zeit-Diagramm eines gemäß der US 6,558,621 B1 durchgeführten Verfahrens; und
- Figur 5b: im Vergleich zum Verfahren gemäß Figur 5a ein schematisches Druck-Zeit-Diagramm eines erfindungsgemäßen Verfahrens.

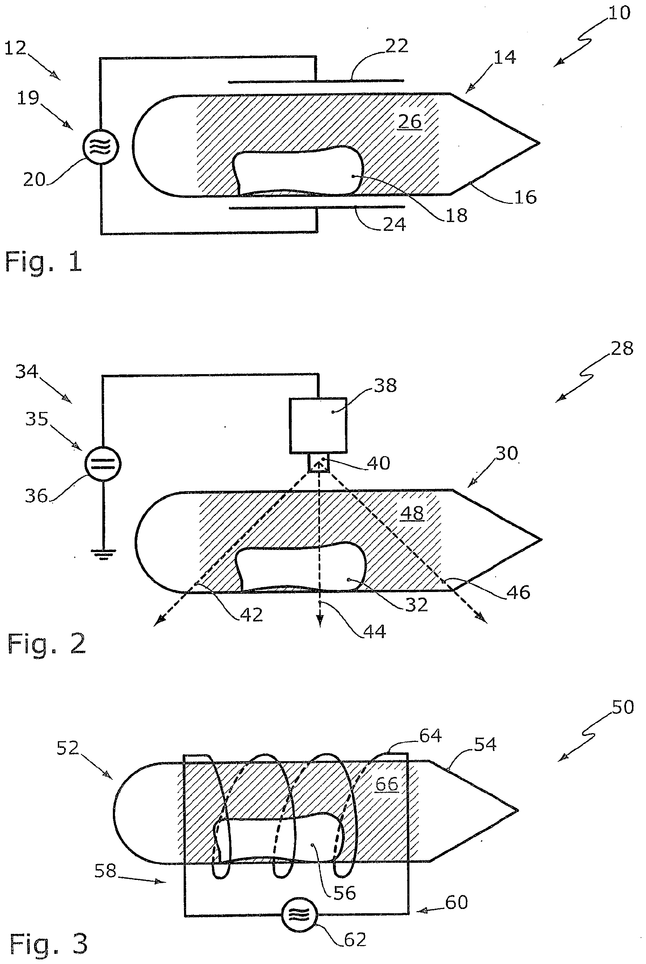

Figur 1 zeigt eine erste Plasmaanlage 10. Die erste Plasmaanlage 10 weist eine Plasmaeinheit 12 und ein Gefäß 14 auf. Das Gefäß 14 ist als elektrodenfreie, unabhängig von der Plasmaeinheit 12 transportierbare Einheit ausgebildet. Das Gefäß 14 umfasst einen Gefäßkörper 16 aus Glas. Der Gefäßkörper 16 schließt einen Gegenstand 18 ein. Der Gegenstand 18 liegt in Form eines Zahnimplantates vor.

Der Gegenstand 18 wurde nach dessen Herstellung in das Gefäß 14 gegeben. Anschließend wurde im Gefäß 14 ein Unterdruck erzeugt und das Gefäß 14 luftdicht verschlossen. Der Gefäßkörper 16 ist nach Aufnahme des Gegenstandes, d.h. in dem in Figur 1 gezeigten Zustand, nicht zerstörungsfrei öffenbar. Hierzu ist der Gefäßkörper 16 in Form einer Glasampulle ausgebildet. Das Gefäß 14 kann dadurch vom Hersteller des Gegenstandes 18 zum Anwender, beispielsweise zu einer Zahnarztpraxis, geliefert werden.

Der Anwender bringt das Gefäß 14 in die Nähe der Plasmaeinheit 12. Die Plasmaeinheit 12 umfasst eine elektrische Plasmaquelle 19, die im vorliegenden Fall einen Hochfrequenzgenerator 20 aufweist. Der Hochfrequenzgenerator 20 ist mit Elektroden 22, 24 der elektrischen Plasmaquelle 19 elektrisch verbunden. Durch Einschalten der elektrischen Plasmaquelle 19 wird in dem Gefäß 14 im Bereich der Elektroden 22, 24 ein Plasma 26 gezündet. Durch das Plasma 26 wird der Gegenstand 18 plasmabehandelt. Im vorliegenden Fall ist das Gefäß 14 mit Inertgas und Sauerstoff gefüllt, so dass die Oberfläche des Gegenstandes 18 von Keimen befreit und aktiviert wird.

Nach der Plasmabehandlung kann der Anwender den Gefäßkörper 16 zerbrechen, den Gegenstand 18 entnehmen und verwenden.

Die Plasmaeinheit 12 ist konstruktiv besonders einfach ausgebildet, wodurch die Herstellungskosten durch die Plasmaeinheit 12 um ca. eine Größenordnung unter den Herstellungskosten für eine "gewöhnliche" Plasmaeinheit mit abpumpbarer Behandlungskammer liegen. Weiterhin müssen der Plasmaeinheit 12 keine Medien, insbesondere keine Gase zu- und/oder abgeführt werden. Die erfindungsgemäße Plasmaeinheit 12 ist dadurch besonders einfach auch durch nicht speziell geschultes Personal verwendbar.

Figur 2 zeigt eine zweite Plasmaanlage 28. Ein Gefäß 30 mit einem Gegenstand 32 sind identisch zu dem Gefäß 14 bzw. dem Gegenstand 18 gemäß Figur 1 ausgebildet. Im Gegensatz zur Plasmaeinheit 12 gemäß Figur 1 weist die Plasmaanlage 28 gemäß Figur 2 jedoch eine Plasmaeinheit 34 auf, deren elektrische Plasmaquelle 35 einen Gleichspannungsgenerator 36 umfasst. Der Gleichspannungsgenerator 36 ist mit einem Magnetron 38 der elektrischen Plasmaquelle 35 elektrisch verbunden.

An dem Magnetron 38 befindet sich eine Antenne 40 zur Abstrahlung elektromagnetischer Strahlung, die in Figur 2 durch Pfeile 42, 44, 46 angedeutet ist. Die elektromagnetischen Wellen bzw. die elektromagnetische Strahlung liegt vorzugsweise im Mikrowellenbereich vor. Die elektromagnetische Strahlung ist dabei so gewählt, dass in dem Gefäß 30 ein Plasma 48 gezündet wird. Das Gefäß 30 ist als unabhängig von der Plasmaeinheit 34 transportierbare Einheit ausgebildet.

Figur 3 zeigt eine dritte Plasmaanlage 50. Die dritte Plasmaanlage 50 weist ein elektrodenfrei ausgebildetes Gefäß 52 auf, das einen Gefäßkörper 54 aus Glas umfasst, wobei in dem Gefäßkörper 54 ein Gegenstand 56 eingebracht ist.

Die dritte Plasmaanlage 50 umfasst zusätzlich zu dem Gefäß 52 eine Plasmaeinheit 58. Das Gefäß 52 ist als unabhängig von der Plasmaeinheit 58 transportierbare Einheit ausgebildet. Die Plasmaeinheit 58 weist eine elektrische Plasmaquelle 60 auf, die einen Hochfrequenzgenerator 62 umfasst. Der Hochfrequenzgenerator 62 ist elektrisch mit einer Induktionsspule 64 zum Zünden eines Plasmas 66 verbunden.

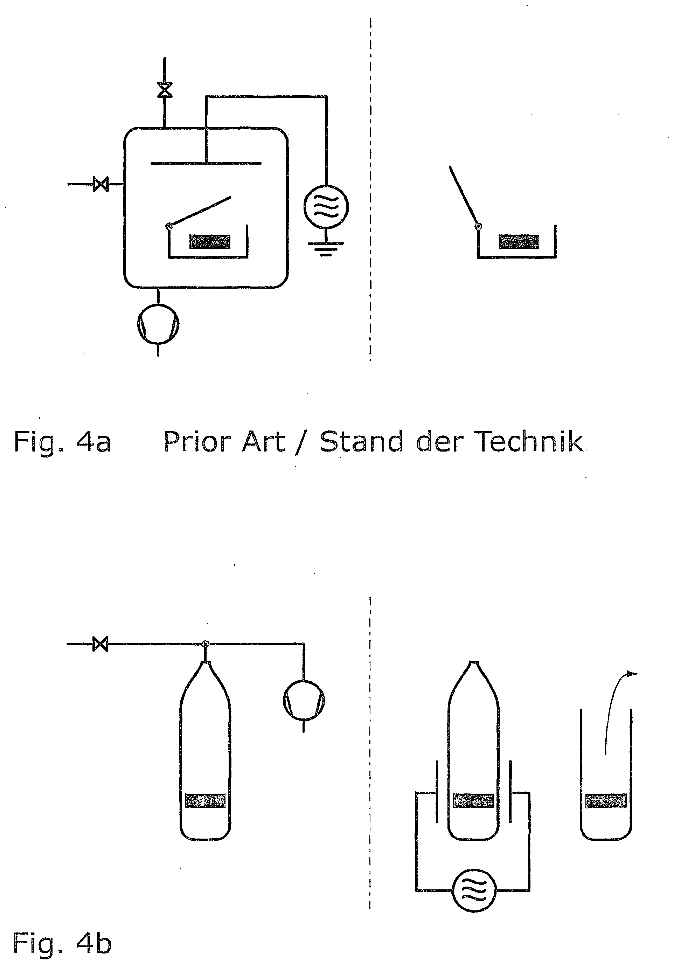

Figur 4a zeigt eine Plasmaanlage gemäß der US 6,558,621 B1. Figur 4a ist in eine Implantatherstellerseite links der strichpunktierten Linie und eine Implantatkundenseite rechts der strichpunktierten Linie unterteilt. Aus Figur 4a ist ersichtlich, dass ein Implantat beim Implantathersteller bei geöffnetem Gefäß plasmabehandelt wird. Nach der Plasmabehandlung wird das Gefäß verschlossen, zum Kunden gebracht und dort vor dem Implantieren geöffnet.

Figur 4b zeigt demgegenüber eine erfindungsgemäße Plasmaanlage. Links der strichpunktierten Linie ist wieder die Implantatherstellerseite und rechts der strichpunktierten Linie die Implantatkundenseite dargestellt. Aus Figur 4b ist ersichtlich, dass das Implantat beim Hersteller in das Gefäß gegeben und das Gefäß mit einer geeigneten Atmosphäre versehen und verschlossen wird. Die eigentliche Plasmabehandlung erfolgt dann beim Kunden, der das Gefäß nach der Plasmabehandlung öffnet und das Implantat implantiert. Im Gegensatz zum Stand der Technik kann somit eine Plasmabehandlung eines Implantates direkt beim Kunden, d.h. in der Arztpraxis bzw. Klinik erfolgen, ohne dass beim Kunden eine aufwändig zu bedienende Plasmaanlage bereitgestellt werden muss.

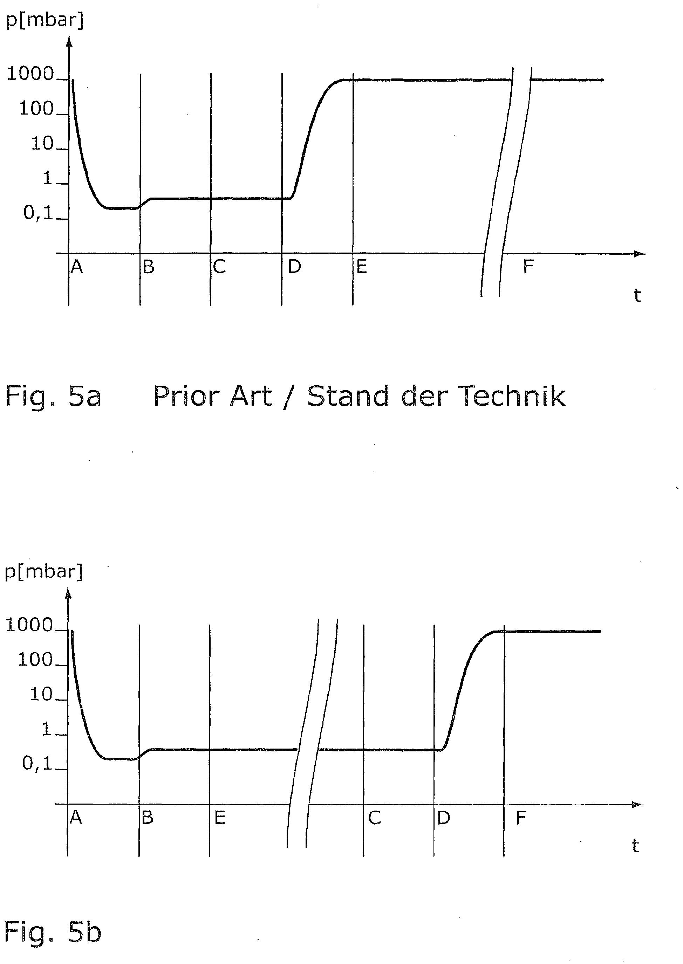

Figur 5a zeigt ein Zeit (X-Achse) - Druck (Y-Achse) - Diagramm eines Verfahrens gemäß der US 6,558,621 B1. Gemäß dem Stand der Technik wird zunächst die Plasmakammer abgepumpt (A), danach erfolgt eine Gasstabilisierung (B), das Zünden eines Plasmas (C), Belüften der Plasmakammer (D), Verpacken/Transportieren/Lagern (E) und Implantieren (F). Aus Figur 5a wird ersichtlich, dass eine lange Zeitspanne zwischen der Plasmabehandlung und dem Implantieren liegen kann, wenn die Plasmaanlage nicht unmittelbar beim Kunden aufgebaut ist.

Figur 5b zeigt demgegenüber ein Zeit-Druck-Diagramm eines erfindungsgemäßen Verfahrens. Hier erfolgt im Gefäß zunächst ein Abpumpen (A), gefolgt von einer Gasstabilisierung (B). Dann wird das Implantat verpackt/transportiert/gelagert (E). Die Plasmabehandlung (C) erfolgt unmittelbar in der Plasmaeinheit beim Kunden. Da die Plasmaeinheit nur elektrischer Anschlüsse aufweist, ist sie für den Kunden sehr einfach zu bedienen. In der Regel weist sie lediglich einen Aus- und Einschalter auf. Unmittelbar nach der Plasmabehandlung erfolgt das Belüften (D) durch Öffnen des Gefäßes und das Implantieren (F).

Durch die Erfindung kann somit sowohl die Plasmabehandlung zeitlich unmittelbar vor dem Implantieren als auch durch eine konstruktiv einfachst ausgebildete Plasmaanlage erfolgen.

Zusammenfassend betrifft die Erfindung eine Plasmaanlage. Die Plasmaanlage besteht aus zumindest zwei separaten Teilen, nämlich einem Gefäß für den zu behandelnden Gegenstand und einer Plasmaeinheit mit einer elektrischen Plasmaquelle zur Zündung eines Plasmas in dem Gefäß. Die Plasmaeinheit weist keine weitere Baugruppe für andere Medien auf, so dass die Plasmaeinheit konstruktiv besonders einfach ausgebildet und leicht bedient werden kann. Besonders bevorzugt umfasst das Gefäß einen Gefäßkörper aus Glas. Der Gefäßkörper ist vorzugsweise einteilig ausgebildet und umschließt den Gegenstand vollständig. Das Plasma kann distanziert von der Plasmaeinheit mittels eines Hochfrequenzgenerators, einer Induktionsspule und/oder eines Magnetrons der elektrischen Plasmaquelle gezündet werden.

## Patentansprüche

1. Plasmaanlage (10, 28, 50) zur Behandlung eines Gegenstandes (18, 32, 56) in Form eines Implantates, wobei die Plasmaanlage (10, 28, 50) ein verschließbares Gefäß (14, 30, 52) zur Aufnahme des Gegenstandes (18, 32, 56) und eine Plasmaeinheit (12, 34, 58) mit einer elektrischen Plasmaquelle (19, 35, 60) zur Erzeugung eines Plasmas (26, 48, 66) in dem Gefäß (14, 30; 52) aufweist, wobei das Gefäß (14, 30, 52) als von der Plasmaeinheit (12, 34, 60) unabhängig transportierbare Einheit ausgebildet ist, wobei das Plasma (26, 48, 66) in dem Gefäß (14, 30, 52) unmittelbar durch die elektrische Plasmaquelle (19, 35, 60) der Plasmaeinheit (12, 34, 58) erzeugbar ist und das Gefäß (14, 30, 52) einen Gefäßkörper (16, 54) aufweist, der im Wesentlichen aus einem dielektrischen Material ausgebildet ist, wobei die Plasmaeinheit (12, 34, 60) keinen Gasanschluss und keine Pumpe aufweist, **dadurch gekennzeichnet, dass** die Plasmaeinheit (12, 34, 60) derart ausgebildet ist, dass das Plasma (26, 48, 66) zündbar ist, wenn sich das Gefäß (14, 30, 52) distanziert zur Plasmaeinheit (12, 34, 60) befindet, wobei der Gefäßkörper (16, 54) zumindest teilweise aus Glas und in Form einer Brechampulle ausgebildet ist.

2. Plasmaanlage nach Anspruch 1, wobei der Gasdruck im Gefäß (14, 30, 52) weniger als 1 bar beträgt.

3. Plasmaanlage nach einem der Ansprüche 1 oder 2, wobei der Gasdruck im Gefäß im Wesentlichen dem Atmosphärendruck entspricht und das Gefäß zu mehr als 40% mit Helium gefüllt ist.

4. Plasmaanlage nach einem der vorhergehenden Ansprüche, wobei die elektrische Plasmaquelle (19) einen Hochfrequenzgenerator (20) und elektrisch mit dem Hochfrequenzgenerator (20) verbundene Elektroden (22, 24) aufweist, zwischen die das Gefäß (14) zumindest teilweise einbringbar ist.

5. Plasmaanlage nach einem der vorhergehenden Ansprüche, wobei die elektrische Plasmaquelle (35) ein Magnetron (38) aufweist und das Gefäß (30) zumindest teilweise von der elektromagnetischen Strahlung des Magnetrons (38) durchdringbar ist.

6. Plasmaanlage nach einem der vorhergehenden Ansprüche, **dadurch gekennzeichnet, dass** die elektrische Plasmaquelle (60) eine Induktionsspule (64) aufweist, in die das Gefäß (52) zumindest teilweise einbringbar ist.

7. Verfahren zur Behandlung eines Gegenstandes (18, 32, 56) in Form eines Implantates, mit den Verfahrensschritten:
a) Einlegen des Gegenstandes (18, 32, 56) in ein Gefäß (14, 30, 52) mit einem Gefäßkörper (16, 54), wobei der Gefäßkörper (16, 54) zumindest teilweise aus Glas und in Form einer Brechampulle ausgebildet ist;
b) Einstellen des Gasdrucks und/oder der Gaszusammensetzung in dem Gefäß (14, 30, 52);
c) Verschließen des Gefäßes (14, 30, 52);
d) Transportieren des Gefäßes (14, 30, 52) zu einer Plasmaeinheit (12, 34, 58);
e) Unmittelbares Zünden eines Plasmas (26, 48, 66) in dem Gefäß (14, 30, 52) mittels einer elektrischen Plasmaquelle (19, 35, 60) der Plasmaeinheit (12, 34, 58) wobei die Plasmaeinheit (12, 34, 58) keinen Gasanschluss und keine Pumpe aufweist und das Gefäß (14, 30, 52) von der Plasmaeinheit (12, 34, 58) distanziert ist.

8. Verwendung eines elektrodenfrei ausgebildeten, unabhängig von einer Plasmaeinheit (12, 34, 58) transportierbaren Gefäßes (14, 30, 52), das zumindest teilweise aus Glas besteht und in Form einer Brechampulle ausgebildet ist, zur Plasmabehandlung eines Gegenstandes (18, 32, 56) in einer Plasmaeinheit (12, 34, 58) nach Anspruch 1, wobei die Plasmaeinheit (12, 34, 58) keinen Gasanschluss und keine Pumpe aufweist.

## Claims

1. Plasma installation (10, 28, 50) for processing an object (18, 32, 56) in the form of an implant, wherein the plasma installation (10, 28, 50) has a closable vessel (14, 30, 52) for receiving the object (18, 32, 56) and a plasma unit (12, 34, 58) having an electrical plasma source (19, 35, 60) for producing a plasma (26, 48, 66) in the vessel (14, 30; 52), wherein the vessel (14, 30, 52) is constructed as a unit which can be transported independently of the plasma unit (12, 34, 60), wherein the plasma (26, 48, 66) can be produced in the vessel (14, 30, 52) directly by the electrical plasma source (19, 35, 60) of the plasma unit (12, 34, 58) and the vessel (14, 30, 52) has a vessel body (16, 54) which is constructed substantially from a dielectric material, wherein the plasma unit (12, 34, 60) does not have a gas connection nor a pump, **characterized in that** the plasma unit (12, 34, 60) is constructed in such a manner that the plasma (26, 48, 66) can be ignited when the vessel (14, 30, 52) is located with spacing from the plasma unit (12, 34, 60), wherein the vessel body (16, 54) is at least partially constructed from glass and in the form of a breakable ampoule.

2. Plasma installation according to claim 1, wherein the gas pressure in the vessel (14, 30, 52) is less than 1 bar.

3. Plasma installation according to any one of claims 1 or 2, wherein the gas pressure in the vessel substantially corresponds to atmospheric pressure and the vessel is filled with helium to a level greater than 40%.

4. Plasma installation according to any one of the preceding claims, wherein the electrical plasma source (19) has a high-frequency generator (20) and electrodes (22, 24) which are electrically connected to the high-frequency generator (20) and between which the vessel (14) can be at least partially introduced.

5. Plasma installation according to any one of the preceding claims, wherein the electrical plasma source (35) has a magnetron (38) and the electromagnetic radiation of the magnetron (38) can pass at least partially through the vessel (30).

6. Plasma installation according to any one of the preceding claims, **characterised in that** the electrical plasma source (60) has an induction coil (64) in which the vessel (52) can be at least partially introduced.

7. Method for processing an object (18, 32, 56) in the form of an implant, having the method steps of:
a) placing the object (18, 32, 56) in a vessel (14, 30, 52) with a vessel body (16, 54), wherein the vessel body (16, 54) is at least partially constructed from glass and designed in the form of a breakable ampoule;
b) adjusting the gas pressure and/or the gas composition in the vessel (14, 30, 52);
c) closing the vessel (14, 30, 52);
d) transporting the vessel (14, 30, 52) to a plasma unit (12, 34, 58);
e) directly igniting a plasma (26, 48, 66) in the vessel (14, 30, 52) by means of an electrical plasma source (19, 35, 60) of the plasma unit (12, 34, 58), wherein the plasma unit (12, 34, 58) does not have a gas connection nor a pump, and the vessel (14, 30, 52) is spaced apart from the plasma unit (12, 34, 58).

8. Use of a vessel (14, 30, 52) which is constructed without electrodes and which can be transported independently of a plasma unit (12, 34, 58) and which at least partially consists of glass and is designed in the form of a breakable ampoule, for plasma processing of an object (18, 32, 56) in a plasma unit (12, 34, 58) according to claim 1, wherein the plasma unit (12, 34, 58) does not have a gas connection nor a pump.

## Revendications

1. Installation à plasma (10, 28, 50) pour le traitement d'un objet (18, 32, 56) sous la forme d'un implant, laquelle installation à plasma (10, 28, 50) présente un récipient fermable (14, 30, 52) destiné à recevoir l'objet (18, 32, 56) et une unité à plasma (12, 34, 58) avec une source de plasma électrique (19, 35, 60) pour produire un plasma (26, 48, 66) dans le récipient (14, 30 ; 52), le récipient (14, 30, 52) étant conçu sous la forme d'une unité transportable indépendamment de l'unité à plasma (12, 34, 60), le plasma (26, 48, 66) pouvant être produit dans le récipient (14, 30, 52) directement par la source de plasma électrique (19, 35, 60) de l'unité à plasma (12, 34, 58) et le récipient (14, 30, 52) présentant un corps de récipient (16, 54) qui est réalisé sensiblement dans un matériau diélectrique, l'unité à plasma (12, 34, 60) ne présentant aucun raccordement de gaz et aucune pompe, **caractérisée en ce que** l'unité à plasma (12, 34, 60) est conçue de façon que le plasma (26, 48, 66) puisse être allumé lorsque le récipient (14, 30, 52) est espacé de l'unité à plasma (12, 34, 60), le corps de récipient (16, 54) étant réalisé au moins en partie en verre et sous la forme d'une ampoule autocassable.

2. Installation à plasma selon la revendication 1, dans laquelle la pression de gaz dans le récipient (14, 30, 52) est inférieure à 1 bar.

3. Installation à plasma selon l'une des revendications 1 ou 2, dans laquelle la pression de gaz dans le récipient correspond sensiblement à la pression atmosphérique et le récipient est rempli à plus de 40 % d'hélium.

4. Installation à plasma selon l'une des revendications précédentes, dans laquelle la source de plasma électrique (19) présente un générateur de hautes fréquences (20) et des électrodes (22, 24) reliées électriquement au générateur de hautes fréquences (20), entre lesquelles le récipient (14) peut être introduit au moins en partie.

5. Installation à plasma selon l'une des revendications précédentes, dans laquelle la source de plasma électrique (35) présente un magnétron (38) et le récipient (30) peut être traversé au moins en partie par le rayonnement électromagnétique du magnétron (38).

6. Installation à plasma selon l'une des revendications précédentes, **caractérisée en ce que** la source de plasma électrique (60) présente une bobine d'induction (64) dans laquelle le récipient (52) peut être introduit au moins en partie.

7. Procédé de traitement d'un objet (18, 32, 56) sous la forme d'un implant, comprenant les étapes suivantes :
a) insertion de l'objet (18, 32, 56) dans un récipient (14, 30, 52) présentant un corps de récipient (16, 54), le corps de récipient (16, 54) étant réalisé au moins en partie en verre et sous la forme d'une ampoule autocassable ;
b) réglage de la pression de gaz et/ou de la composition de gaz dans le récipient (14, 30, 52) ;
c) fermeture du récipient (14, 30, 52) ;
d) transport du récipient (14, 30, 52) vers une unité à plasma (12, 34, 58) ;
e) allumage direct d'un plasma (26, 48, 66) dans le récipient (14, 30, 52) au moyen d'une source de plasma électrique (19, 35, 60) de l'unité à plasma (12, 34, 58), l'unité à plasma (12, 34, 58) ne présentant aucun raccordement de gaz et aucune pompe et le récipient (14, 30, 52) étant espacé de l'unité à plasma (12, 34, 58).

8. Utilisation d'un récipient (14, 30, 52) réalisé sans électrodes, transportable indépendamment d'une unité à plasma (12, 34, 58), qui est composé au moins en partie de verre et réalisé sous la forme d'une ampoule autocassable, pour le traitement au plasma d'un objet (18, 32, 56) dans une unité à plasma (12, 34, 58) selon la revendication 1, l'unité à plasma (12, 34, 58) ne présentant aucun raccordement de gaz et aucune pompe.
